# EUROPEAN PATENT APPLICATION

(11) **EP 4 763 199 A1**
(43) Date of publication of application: **24.06.2026**
(21) Application number: 25712645.8
(22) Date of filing: 03.02.2025
(51) Int. Cl.: A61K 31/05, A61K 31/19, A61K 31/191, A61K 31/7052, A61K 31/7068, A61K 31/7072, A61K 31/7076, A61K 31/708, A61K 31/7105, A61K 31/711, A61K 31/716, A61K 31/734, A61K 31/737, A61K 36/63, A61K 45/06

(54) **COMBINED PREPARATION OF NUCLEBASES AND A MIXTURE OF TRITERPENIC ACID AND POLYPHENOLS FOR USE THEREOF IN IMPROVING IMMUNITY**

(30) Priority: 15.02.2024 ES 202430116
(71) Applicant: Bioiberica, S.A.U., 08389 Palafolls Barcelona (ES)
(72) Inventor: VELASCO ÁLVAREZ, Javier, 08389 Palafolls Barcelona (ES); SEGARRA LÓPEZ, Sergi, 08389 Palafolls Barcelona (ES); MARTÍNEZ PUIG, Daniel, 08389 Palafolls Barcelona (ES)
(74) Representative: Pons IP
(86) International application number: PCT/ES2025/070051
(87) International publication number: WO 2025/172628

(57) **Abstract**

The present invention relates to pharmaceutical and food compositions comprising nucleobases and/or sources of nucleobases and a mixture of triterpenic acid and polyphenols. Preferably, the compositions include Nucleoforce^{®} and Aquolive^{®}. Said compositions are useful in the treatment and/or prevention of bacterial infection; as well as being useful in improving immune function.

## Description

The present invention relates to pharmaceutical and food compositions comprising nucleobases and/or sources of nucleobases, and a mixture of triterpenic acid and polyphenols. Preferably, the compositions include Nucleoforce^{®} and Aquolive^{®}. Said compositions are useful in the treatment and/or prevention of bacterial infection; as well as being useful in stimulating immune function. Therefore, the present invention falls within the fields of biotechnology, pharmacy and food, more particularly within compositions intended for the prevention and/or treatment of bacterial infection, and improving the immunity of a subject.

### STATE OF THE ART

Whiteleg shrimp, *Litopenaeus vannamei,* is one of the largest cultivated tropical shrimp species in the world, native to the Pacific Ocean. Shrimp are characterised by rapid growth, high tolerance to a wide range of water salinities (5 to 40 ppt), high intensification, low protein requirements and other characteristics suitable for super-intensive aquaculture. However, environmental factors such as water temperature, salinity fluctuation, sudden and rapid changes in pH, low dissolved oxygen and toxins such as ammonia, nitrite, hydrogen sulphide and heavy metals, affect shrimp growth and survival rates.

In cases of stress, the risk of opportunistic infections increases, especially those related to the respiratory and digestive systems. A clear example of stress-related infections at an early age comes from animal production. In commercially reared animals these infections are particularly relevant.

Global progress in shrimp farming is facing increasing environmental and pathological challenges, in addition to a low cold water tolerance capacity in most shrimp producing countries. To that end, enhancing shrimp immunity is an important direction in current support policies to prevent their diseases.

There is growing concern about the overuse of antibiotics to combat opportunistic infections due to the emergence of antimicrobial resistance. Thus, the use of natural immunostimulants is recommended as a healthier and safer therapy than the administration of antibiotics and/or vaccines to control aquaculture pathogens.

It has been disclosed that ingredients in dietary supplements can improve immune function and reduce the risk of infectious diseases. Some studies suggest that supplementation with multiple micronutrients that support the immune system is beneficial, but more research is needed (Gombart, et al. Nutrients, 6;12(1):236. 2020*).*

Thus, there are other isolated nutritional compounds, and the alleged benefits of such compounds include, among numerous other effects, immune support, anti-inflammatory effects, anti-ageing effects, cardiac support and digestive support. Unfortunately, there is only a fairly small body of evidence to support some aspects of these purported benefits when these vitamins and other isolated nutritional compounds are ingested.

In view of the foregoing, novel approaches and strategies for managing bacterial infections are required, and in general, for improving the immunity of a subject.

### DESCRIPTION OF THE INVENTION

By carrying out tests aimed at improving crustacean farming performance, the inventors have observed that the combined administration of a nucleobase (or a source of nucleobases), and a mixture of triterpenic acid and polyphenols, as a food supplement, improves the immunity of the subjects, by promoting both the prevention and treatment of a bacterial infection.

In the examples of the present description, it is demonstrated by using in vivo models that the combined preparation defined in the claims synergistically improves growth performance at the production level (Example 1) and the ability to tolerate bacterial infection (Example 2).

Thus, in a first aspect, the present invention relates to a combined preparation, hereinafter "combined preparation of the invention", comprising:
a) A first composition comprising a nucleobase and/or a source of nucleobases selected from the group consisting of nucleosides, nucleotides, RNA, DNA, and combinations thereof, and
b) A second composition comprising a mixture of triterpenic acid and polyphenols,
wherein the concentration of the first composition relative to the second composition is between 250 and 750 ppm.

The term "combined preparation" relates to a preparation in which there is more than one composition, in this case the first defined composition a) and the second defined composition b). In the combined preparation of the invention, both compositions need not be present as a bond in order to be available, but rather can be separated so that they can be applied separately, either simultaneously, sequentially, or mixed in a single solution. In this way, it does not necessarily result in a true combination, in view of the possible physical separation of the two compositions. Preferably, the first and second composition within the combined preparation are administered simultaneously.

The combined preparation of the invention comprises a first composition a) comprising a nucleobase and/or a source of nucleobases selected from the group consisting of nucleosides, nucleotides, RNA and DNA.

As used in the present invention, nucleobases are cyclic organic compounds that include two or more nitrogen atoms. They are an essential part of nucleosides, nucleotides and nucleic acids. The most preferred nucleobases, which form part of the first composition of the present invention, are purine (adenine and guanine) and pyrimidine (cytosine, thymine and uracil) bases.

The term "source of nucleobases" relates to nucleosides, nucleotides, RNA (ribonucleic acid), DNA (deoxyribonucleic acid) or equivalent.

Examples of nucleosides include, but are not limited to, ribose nucleosides, such as adenosine, guanosine, uridine and cytidine; and deoxyribose nucleoside, such as deoxyadenosine, deoxyguanosine, deoxycytidine and deoxyuridine.

Examples of nucleotides include, but are not limited to, phosphate esters of mononucleosides, such as adenosine monophosphates (AMP), guanosine (GMP), uridine (UMP), cytidine (CMP), deoxythymidine (dTMP) and deoxycytidine (dCMP); and di- and trinucleoside phosphates, such as uridine diphosphate (UDP) and uridine triphosphate (UTP).

The origin of the source of nucleobases is, preferably, yeast, meat or similar products.

As understood by a person skilled in the art, within the first composition of the combined preparation of the invention, combinations of sources of nucleobases are also contemplated. Thus, the first composition can comprise, simultaneously, two or more of said sources such as nucleosides, nucleotides, RNA and DNA. In a preferred embodiment of the combined preparation of the invention, the first composition comprises uridine monophosphate, adenosine monophosphate, cytidine monophosphate and guanine-5'-monophosphate-disodium.

In another preferred embodiment of the combined preparation of the invention, alone or in combination with any and all of the above preferred embodiments, the first composition comprises between 8 and 9 % of uridine monophosphate, between 7 and 8 % of adenosine monophosphate, between 4 and 5 % of cytidine monophosphate and between 8 and 9 % of guanine-5'-monophosphate-disodium (% by weight of the total weight of the first composition). All the ranges mentioned in this description include the ends of the range.

In another preferred embodiment of the combined preparation of the invention, alone or in combination with any and all of the above preferred embodiments, the first composition is Nucleoforce^{®}. The Nucleoforce^{®} composition is a solution the main components of which are free nucleotides, such as but are not limited to, 8.3 % of uridine monophosphate, 7.45 % of adenosine monophosphate, 4.83 % of cytidine monophosphate and 8.27 % of guanine-5'-monophosphate-disodium (% by weight of the total weight of the first composition). Likewise, Nucleoforce^{®} can also comprise active precursors.

Nucleoforce^{®} can be obtained from extraction, purification and hydrolysis of RNA from *Saccharomyces cerevisiae.* This composition is marketed by Bioiberica S.A.U. (www.bioiberica.com) and can be purchased by the person skilled in the art through the usual sales channels.

The components of the first composition of the combined preparation of the invention can be in any amount/concentration, and said amounts can vary according to the nutritional needs of the subject for whom the composition of the invention is intended.

The person skilled in the art knows how to adapt the amounts of the different components of the first composition of the invention according to said nutritional needs.

The combined preparation of the invention comprises a second composition b) comprising a mixture of triterpenic acid and polyphenols, wherein the concentration of the first composition relative to the second composition is between 350 and 650 ppm.

"Triterpenic acid" is an organic compound derived from isoprene, which is pharmacologically very efficient, that mainly, is of plant origin and is widely spread in nature. Triterpenic acids occur predominantly as pentacyclic compounds. Examples of triterpenic acids, include, but are not limited to, chorosolic, oleanolic, maslinic, Asian, madecassic, betulinic, glycyrrhetinic, ursolic and boswellic acids. **In** the context of the present invention, the second composition of the combined preparation of the invention can comprise a triterpenic acid and/or a derivative of a triterpenic acid. Examples of compounds derived from triterpenic acids are very well-known in the state of the art.

In the present invention, "polyphenols" is understood as compounds having a molecular structure characterised by the presence of one or more phenolic rings. They originate mainly from plants, which synthesise them in large amounts, as a by-product of the secondary metabolism thereof. Some are indispensable for plant physiological functions. Others are involved in defence functions in situations of stress and various stimuli (hydric, luminous, etc.). Likewise, it is a group of substances with a high antioxidant capacity and positive effects on human health.

Examples of polyphenols, include, but not limited to phenolic acids, stilbenes, lignans, phenolic alcohols, hydroxytyrosol, oleuropein and flavonoids.

Both triterpenic acid and polyphenols are active compounds obtainable from plants. In the present invention, "active substance" or "bioactive compound" is understood as a compound that influences the cellular and physiological activities of a subject, thus having a beneficial effect on their growth and health. Chemically, these compounds are of diverse origin and act through different mechanisms of action. Thus, there are carotenoids, polyphenols, terpenes, lignans, organosulphur compounds, glucosilonates, saponins, marine polysaccharides, etc. In another preferred embodiment of the combined preparation of the invention, alone or in combination with any or all of the above preferred embodiments, the triterpenic acid and polyphenols of the second composition are obtained from a plant extract, preferably, of the Olea genus, more preferably, *Olea europaea.* Preferably, the mixture of triterpenic acid and polyphenols means, at least, 5 %, 6 %, 7 %, 8 %, 9 %, 10 %, 11 %, 12 %, 13 %, 14 % or 15 % by weight of the total of the second composition.

The second composition of the combined preparation of the invention can comprise fulvic acids. "Fulvic acids" are a mixture of weak aliphatic and aromatic organic acids that dissolve in water at all pH levels (acids, neutral and alkaline), which is produced during the decay of organic matter. It can be found on the ground, marine sediments, in waste water and compost. In another preferred embodiment of the combined preparation of the invention, alone or in combination with any or all of the above preferred embodiments, the second composition further comprises fulvic acids.

In another preferred embodiment of the combined preparation of the invention, the second composition is Aquolive^{®}. The Aquolive^{®} composition is commercially available and can be purchased by the person skilled in the art through the usual sales channels.

In another preferred embodiment of the combined preparation of the invention, the first composition is the commercial product Nucleoforce^{®} and the second composition is the commercial product Aquolive^{®}.

The inventors of the present invention have tested different concentrations of the first and the second composition within the combined preparation to obtain the optimum concentrations of each composition to exert a greater effect (greater than the effect that would be obtained by adding the effects of each of the two compositions when used separately). Thus, in the combined preparation of the invention, the concentration of the first composition relative to the second composition is between 250 and 750 ppm (including range ends). In the present invention, ppm or part per million (10⁻⁶) is understood as the number of units of a given substance (agent, etc.) per million units of the whole.

The first composition of the combined preparation of the invention can be at any concentration within the stated range. However, in a preferred embodiment, the concentration of the first composition relative to the second is between 350 and 650 ppm (including range ends) which, in another, still more preferred embodiment, is between 450 and 550 ppm (including range ends) which, in another, still more preferred embodiment, is 500 ppm.

As understood by a person skilled in the art, the combined preparation of the invention can be formulated in various ways depending on how it is to be administered. Therefore, the combined preparation of the invention can be found in liquid form (solutions), emulsions, suspensions, syrups, etc.), solid form (capsules, pills, powders, etc.) or semisolid form (gels).

The combined preparation of the invention can form part of a food product, or of a pharmaceutical composition.

To that end, another aspect of the present invention relates to a food product, hereinafter "food product of the invention", comprising the combined preparation of the invention. In the context of the present invention, the terms "food product" and "nutritional composition" are considered to be equivalent and can be used interchangeably throughout the present description.

The food product of the invention can be for animal use/consumption, including a mammal and, therefore, the human being, and refers to any product the ingestion of which beneficially affects the subject by positively promoting growth and improving the state of immunological health within the meaning of the present invention.

In a preferred embodiment of the food product of the invention, said food product is a food or a (dietary or nutritional) supplement, an ingredient of a functional food, a medicinal food or a nutraceutical.

Examples of foods include, but are not limited to, plant products, meat products, snacks, beverages, cereals, bakery products, biscuits, dairy products, milks, fermented milks (such as yoghurt or cheese), ice creams, butters, margarines, etc. In another preferred embodiment of the food product of the invention, the food is a feed, a beverage (e.g. milkshakes, juices, sports nutrition drinks, etc.), a dairy product (e.g. milk), yoghurt, cheese, kefir, etc.), a confectionery product, a protein bar, cereals, a cereal bar, a gel or soup.

The term "supplement" refers to, in the context of the present invention, products or preparations the purpose of which is to supplement or complement the normal diet of a subject by providing nutrients or other substances with a physiological and/or nutritional effect. Examples of supplements include, without being limited to, a dietary supplement, a nutritional supplement, a food supplement, etc.

In the context of the present invention, the term "nutraceutical" refers to products of natural origin with biologically active properties, beneficial to health and with defined preventive and/or therapeutic capacity. Examples of nutraceuticals include, without being limited to, folic acid, sterols, soluble fibre, phenolic compounds (flavonoids, anthocyanins, isoflavones, carotenoids, etc.), OMEGA-3 fatty acids (ALA (alpha-linolenic acid), EPA (eicosapentaenoic acid), DHA (docosahexaenoic acid).

By definition, "functional foods" refers to natural food or a food to which a component has been added, or a food from which a component has been removed by technological or biological means; therefore, these are foods which, apart from its nutritional content, contain ingredients that have a specific activity on the physiological functions of the human body, promoting physical capacity and mental fitness. Examples of functional foods include, without being limited to, foods containing certain minerals, amounts of vitamins, fatty acids, fibre and probiotics that have cultures of beneficial live microorganisms. They are usually in the form of foods for daily use enriched in certain nutrients or health-promoting substances.

The main difference between nutraceutical and functional food is that the nutraceutical is made up of the biological part of the functional food, for example, cholesterol-lowering (i.e. phytosterol).

The combined preparation of the invention can form part of a pharmaceutical composition. Thus, in another aspect, the present invention relates to a pharmaceutical composition, hereinafter "pharmaceutical composition of the invention", comprising the combined preparation of the invention.

The pharmaceutical composition of the invention can be administered to an animal, including a mammal and, therefore, the human being, and refers to any composition the administration of which beneficially affects the subject, positively promoting their growth and improving the immune health status in the sense indicated in the present invention.

In a preferred embodiment, the pharmaceutical composition comprises a pharmaceutically acceptable carrier and/or excipient.

The term "excipient" refers to a substance that aids the absorption of any of the components of the composition, in other words, the compositions comprised in the combined preparation of the invention, or that stabilises said components or aids the preparation of the pharmaceutical composition in the sense of giving it consistency or providing flavours that make it more pleasant. Thus, excipients could have the function of holding components together (e.g., starches, sugars or celluloses), to sweeten, to provide a colouring agent, to protect the active ingredient (e.g., to isolate same from the air and/or humidity, that of refilling a tablet, capsule, or any other form of presentation, of disintegration to facilitate the dissolution of the components, etc., without excluding other types of excipients not mentioned herein. Therefore, the term "excipient" is defined as a material that, included in galenic forms, is added to active ingredients or to the associations thereof to allow for the preparation and stability thereof, to modify the organoleptic properties thereof or to determine the physicochemical properties of the pharmaceutical composition and the bioavailability thereof. The "pharmaceutically acceptable" excipient must allow for the activity of the compounds of the pharmaceutical composition, in other words, that is compatible with the compositions comprised in the combined preparation of the invention.

The "galenic form" or "pharmaceutical form" is the disposition to which the active ingredients and excipients are adapted in order to constitute a composition or a drug. It is defined by the combination of the form in which the pharmaceutical composition is presented by the manufacturer and the form in which it is administered.

The "carrier" or "vehicle" is, preferably, an inert substance, and as well as the excipient, its purpose is to facilitate the incorporation of other compounds, allow better dosing and administration and/or give the pharmaceutical composition consistency and shape. Therefore, the carrier is a substance that is used in the drug to dilute any of the components of the pharmaceutical composition of the present invention to a certain volume or weight; or that, even without diluting said components, is able to allow for a better dosage and delivery and/or to give consistency and shape to the drug. When the form of presentation is liquid, the pharmaceutically acceptable carrier is the diluent.

In addition, the excipient and the carrier must be pharmacologically acceptable, in other words, the excipient and the carrier are allowed and evaluated so that no harm is caused to the subject to which it is administered. In each case, the form of presentation of the pharmaceutical composition shall be adapted to the type of administration used. To that end, the composition can be in the form of solutions or any other clinically permitted form of administration and in a therapeutically effective amount. The pharmaceutical composition of the invention can be formulated in solid forms (tablets, powders, microgranules (pellets), tablets, caplets, capsules, granules, pills, patches, etc.), semisolid (gels) or liquid (solutions), suspensions, emulsions, syrups, drops, drinkable vials, etc.). In another preferred embodiment of the pharmaceutical composition of the invention, said composition is formulated for oral administration. The form adapted for oral administration refers to a physical state that can allow for the oral administration thereof.

As the examples below show, the combined preparation of the invention is useful for increasing the growth performance of the subjects. Furthermore, in a situation of simulated bacterial infection *in vivo* (culture under laboratory conditions) there is an unexpected increase in the survival rate of the subjects, which has a positive impact on the ability of the subjects to tolerate an infectious situation, showing a synergistic effect associated with the combined use of the compositions comprised in the combined preparation of the invention. In summary, the combined preparation of the invention has a positive and synergistic effect on bacterial growth and infections.

Therefore, in another aspect, the invention relates to the combined preparation, the food product or the pharmaceutical composition of the invention, for use as a medicinal product or, alternatively, to the use of the combined preparation of the invention, the food product of the invention or the pharmaceutical composition of the invention for manufacturing a medicinal product.

The term "medicinal product" refers to a substance used for prevention, alleviation, treatment or cure of diseases, syndromes or clinical conditions in animals, including human beings. In the context of the present invention, it relates to the combined preparation, the food product or the pharmaceutical composition in a therapeutically effective amount.

In the sense used in this description, the term 'therapeutically effective amount' refers to the amount of the combined preparation, the food product or the pharmaceutical composition of the invention that produces the desired effect. The dose for obtaining a therapeutically effective amount depends on a variety of factors, for example, age, weight, sex or tolerance of the subject.

The medicinal product of the invention can be used either alone or in combination with other medicinal products or compositions to promote or enhance immunity in a subject, and treat and/or prevent a bacterial infection in a subject. The medicinal product of the present invention can be used together with other active ingredients or therapies as a combination therapy. The other active ingredients can be part of the same composition or can be provided by means of a different composition, being administered simultaneously or sequentially.

In another aspect, the invention relates to the combined preparation, the food product or the pharmaceutical composition of the invention, for use in improving immunity in a subject, or alternatively, to the use of the combined preparation of the invention, the food product of the invention or the pharmaceutical composition of the invention for manufacturing a medicinal product for improving immunity in a subject. Similarly, the present invention relates to a method for improving immunity in a subject, comprising administering the combined preparation, the food product or the pharmaceutical composition of the invention to said subject.

In the context of the present invention, the term "improving the immunity of a subject" refers to promoting, increasing, stimulating, increasing or inducing the immune system in a subject, in other words, promoting, increasing, stimulating, increasing or inducing the set of biological elements and processes within the subject (lymphocytes, leukocytes, cytokines, etc.) that allow it to maintain homeostasis or internal equilibrium in the face of external aggressions, whether biological (pathogens) or physico-chemical (such as pollutants or radiation) and internal (e.g., cancer cells).

In another aspect, the invention relates to the combined preparation, the food product or the pharmaceutical composition of the invention, for use in the treatment and/or prevention of a bacterial infection in a subject or, alternatively, to the use of the combined preparation, the food product or the pharmaceutical composition of the invention for manufacturing a medicinal product for the treatment and/or prevention of a bacterial infection in a subject. Similarly, the present invention relates to a method for the treatment and/or prevention of a bacterial infection in a subject, comprising administering the combined preparation, the food product or the pharmaceutical composition of the invention to said subject.

The term "treatment" or "treat" as understood in the present invention, refers to combating the effects caused as a consequence of the disease or pathological condition of interest in a subject, which includes:
(i) inhibiting the disease or pathological condition, in other words, stopping its development;
(ii) alleviating the disease or pathological condition, in other words, causing the remittance of the disease or pathological condition or the symptoms thereof;
(iii) stabilising the disease or pathological condition.

The term "prevention" or "preventing" an infection as understood in the present invention, consists of preventing the onset of the disease or clinical condition, in other words, preventing the disease or pathological condition from appearing in a subject, in particular, when said subject has a predisposition for the pathological condition, but has not yet been diagnosed as suffering from the same.

In a preferred embodiment of the invention, bacterial infection is caused by *Vibrio parahaemolyticus (*also called *V. parahaemolyticus).* The genus *Vibrio,* belonging to the family *Vibrionaceae,* has 48 species, of which, at least 12 are recognised as human pathogens. Among these, *Vibrio cholerae, Vibrio parahaemolyticus* and *Vibrio vulnificus* are the most important pathogenic vibrios, both in terms of disease-causing capacity and the magnitude of the overall disease burden. *V. parahaemolyticus* is a gram-negative bacillus, slightly curved, facultative aerobic, halophilic, oxidase positive, a glucose, but not sucrose, and variable urease fermenter. It requires selective means for its development, with a NaCl concentration of 1 % and is widely distributed in marine environments. This organism is frequently isolated from a variety of raw sea products, particularly seafood.

The term "subject" refers to a human being or a non-human animal, preferably a mammal or arthropod. The subject to which the present invention relates can be, for example, but are not limited to, a ruminant such as sheep, goat or cattle, dromedary, camel, llama, kangaroo, pig; poultry such as turkey, duck, quail, goose, pigeon, chicken, hen or rooster, horse; pets such as a dog or cat; as well as arthropods such as crustaceans or myriapods.

Therefore, in a preferred embodiment of the combined preparation, the food product or the pharmaceutical composition for therapeutic use according to the present invention, the subject is a mammal or an arthropod. In a more preferred embodiment, wherein the mammal is a primate, preferably, a human being. In a still more preferred embodiment, wherein the arthropod is a crustacean, preferably, a caridea, more preferably, a shrimp, a prawn, a baby prawn or a rockpool prawn.

In the present invention, "arthropods" are understood to be invertebrate animals equipped with an external skeleton (exoskeleton), the body divided into articulated segments and appendages, among other characteristics. Arthropods are the most numerous and diverse of the 29 phyla that make up the animal kingdom. Depending on the number of legs they have, arthropods are divided into: insects (three pairs of legs), arachnids (four pairs of legs), crustaceans (five pairs of legs) and myriapods (with more than 10 legs).

### DESCRIPTION OF THE FIGURES

Figure 1. Feed conversion ratio (FCR) after 8 weeks in each of the study groups. Different superscript letters indicate statistically significant differences (p<0.05). NF: Nucleoforce^{®}; AO: Aquolive^{®}.
Figure 2. Evolution of the survival rate (%) in the different study groups over time. Different superscript letters indicate statistically significant differences (p<0.05). NF: Nucleoforce^{®}; AO: Aquolive^{®}; PC: Positive Control; NC: Negative Control.

### EXAMPLES

Next, the invention will be illustrated by means of tests carried out by the inventors, which demonstrate the effectiveness of the product of the invention.

### Combined preparation

The components of the combined preparation of the invention used in the examples of the present patent application are shown in Tables 1 and 2.

| Table 1 - Components of the first composition | | |
|---|---|---|
| | Component | Amount (% by weight) |
| | uridine monophosphate | 8.3 % |
| Nucleoforce^{®} | adenosine monophosphate | 7.45 % |
| | cytidine monophosphate | 4.83 % |
| | guanine-5'-monophosphate-disodium | 8.27 % |

| Table 2 - Components of the first composition | | |
|---|---|---|
| | Component | Amount (% by weight) |
| Aquolive^{®} | Triterpenic acid | 8.0 % |
| | Polyphenols | 2.0 % |

**Example 1.** Study of the effect of the combined preparation of the invention on the productivity of whiteleg shrimp (*Litopenaeus vannamei*) under recirculating aquaculture system (RAS) conditions.

A total of 1250 whiteleg shrimp (*Litopenaeus vannamei*) free of specific pathogens, belonging to a shrimp farm in Vietnam, were acclimatised for 2 days and, subsequently, were classified into 6 treatment groups (250 shrimps per group); 5 replicates (400L tanks) per treatment (see Table 3).

| Table 3. Treatment groups in the productivity study | |
|---|---|
| Treatment group | Description |
| Control | Control diet without supplementation (no Nucleoforce^{®} or Aquolive^{®} is administered) |
| NF1000 | Same control diet supplemented with Nucleoforce^{®} at 1000ppm |
| NF500 | Same control diet supplemented with Nucleoforce^{®} at 500ppm |
| AO500 | Same control diet supplemented with Aquolive^{®} at 500ppm |
| NF500AO500 | Same control diet supplemented with Nucleoforce^{®} at 500ppm and Aquolive^{®} at 500ppm |

Each tank was equipped with an aeration system to maintain oxygen above 6 ppm during the study period. The maximum density was 200 shrimps/m³. The feeding regime was one of small frequent amounts; once every 4 hours for a period of 24 hours (i.e., 6 times per day). The shrimps were fed until satiated. The water temperature was maintained between 28 and 32 °C.

Several common productivity parameters were assessed during the 8-week test:
- Variations in weight and biomass:
- Feed intake
- Feed conversion ratio (FCR)

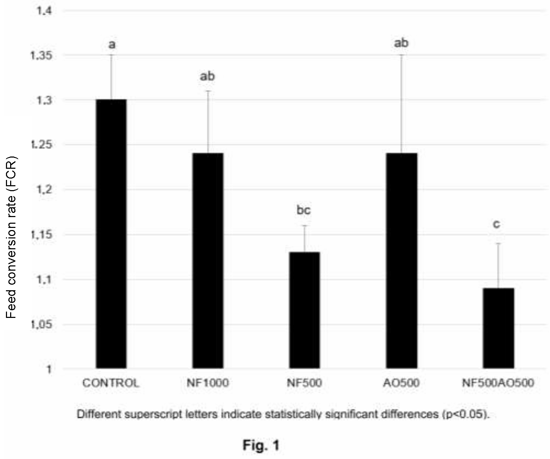

Tables 4 and 5 show the results of feed intake after 8 weeks of treatment in the different groups. Similarly, Figure 1 shows how, despite achieving a numerically better FCR (lower FCR means higher efficiency), AO500 does not achieve a statistically significant difference (p>0.05) compared to the control group. The NF500 group did have a significantly better FCR (p<0.05) than the control group. The most striking result is a very marked improvement of the FCR in the NF500AO500 group, which achieves a statistically significant improvement (p<0.05), both against the control and also against NF1000, NF500 and AO500. In this regard, a synergy at the production level between the Nucleoforce^{®} and Aquolive^{®} components is shown by combining them in a 1:1 ratio.

| Table 4. Productivity after 8 weeks | | | | |
|---|---|---|---|---|
| Treatment group | Average initial weight (g) | Average final weight (g) | Final biomass (g) | Average weight gain (g) |
| Control | 1.23 ± 0.05a | 22.03 ± 4.25a | 858.80 ± 23.59a | 20.80 ± 0.55a |
| NF1000 | 1.22 ± 0.04a | 23.77 ± 4.81b | 908.04 ± 54.66ab | 22.55 ± 0.64b |
| NF500 | 1.21 ± 0.05a | 23.43 ± 4.23b | 988.04 ± 26.71bcd | 22.22 ± 0.89b |
| AO500 | 1.22 ± 0.03a | 23.06 ± 4.90b | 922.44 ± 78.75ab | 21.85 ± 0.64b |
| NF500AO500 | 1.22 ± 0.03a | 23.74 ± 4.77b | 1014.43 ± 21.83cde | 22.51 ± 0.72b |

| Table 5. Productivity after 8 weeks | | | | |
|---|---|---|---|---|
| Treatment group | Average daily gain (g/day) | Specific growth rate (%/day) | Feed consumption (g) | Feed conversion ratio |
| Control | 0.37 ± 0.01a | 5.15 ± 0.09a | 1032.16 ± 10.91a | 1.30 ± 0.05a |
| NF1000 | 0.40 ± 0.01b | 5.31 ± 0.08b | 1045.18 ± 6.80ab | 1.24 ± 0.07ab |
| NF500 | 0.40 ± 0.02b | 5.29 ± 0.11b | 1052.27 ± 0.42bc | 1.13 ± 0.03bc |
| AO500 | 0.39 ± 0.01b | 5.25 ± 0.06ab | 1057.54 ± 5.69ab | 1.24 ± 0.11ab |
| NF500AO500 | 0.40 ± 0.01b | 5.29 ± 0.05b | 1058.80 ± 13.21bc | 1.09 ± 0.05c |

**Example 2**. Study of the effect of the combined preparation of the invention on the survival of whiteleg shrimp (*Litopenaeus vannamei*) against a challenge with *Vibrio parahaemolyticus* under laboratory conditions.

A total of 720 whiteleg shrimps affected by acute hepatopancreatic necrosis, belonging to a shrimp farm in Vietnam, were used to assess the tolerance of subjects to bacterial infection with *V. parahaemolyticus.*

Once on the premises, shrimps were acclimatised connected to an open flow system for 2 days and, subsequently, were classified into 6 treatment groups (120 shrimps per group); 4 replicates (90L tanks) per treatment with 30 shrimps/tank (see Table 6).

| Table 6. Treatment groups in bacterial challenge | | |
|---|---|---|
| Treatment group | | Description |
| No challenge | Negative Control | Control diet |
| With challenge | Positive Control | Control diet without supplementation (no Nucleoforce^{®} or Aquolive^{®} is administered) |
| | NF1000 | Same control diet supplemented with Nucleoforce^{®} at 1000ppm |
| | NF500 | Same control diet supplemented with Nucleoforce^{®} at 500ppm |
| | AO500 | Same control diet supplemented with Aquolive^{®} at 500ppm |
| | NF500AO500 | Same control diet supplemented with Nucleoforce^{®} at 500ppm and Aquolive^{®} at 500ppm |

The water temperature was kept between 24 °C and 30 °C. Each tank was equipped with an aeration system with 2 diffuser stones to keep oxygen above 6 ppm during the study period, a biofilter bucket and covered with a plastic lid to reduce the risk of cross-contamination. The light regime (photoperiod) was 12:12h (Light:Dark). The maximum density was 200 shrimps/m3.

The feeding regime was one of small frequent amounts; once every 4 hours for a period of 24 hours (i.e., 6 times per day). The shrimps were fed until satiated.

The bacterial challenge was carried out as follows: an isolate of *V. parahaemolyticus* was collected from shrimp affected by acute hepatopancreatic necrosis belonging to a shrimp farm in Vietnam. The shrimps were subjected to an immersion challenge. Bacterial suspensions were added to the tanks to achieve the desired density, which was measured by optical density absorbance (OD600 nm), to achieve a lethal dose of 90 % mortality within 10 days of the challenge. Shrimp were followed for 10 days after challenge to quantify and compare the survival rate between groups.

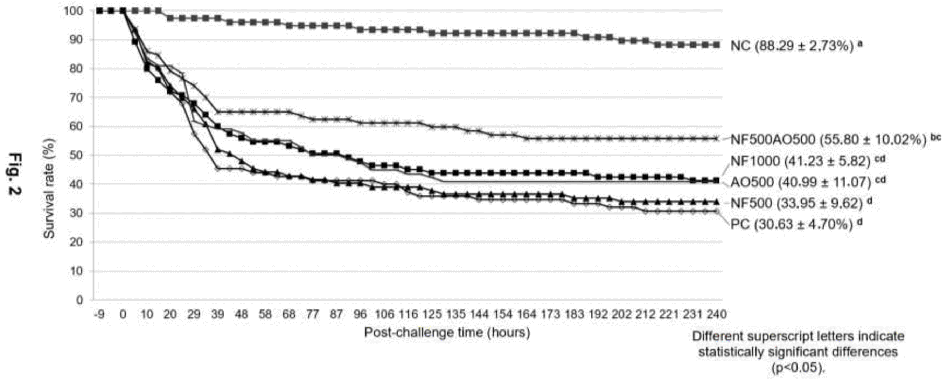

Figure 2 shows the results of the evolution of the survival rate throughout the study in the different groups. The Positive Control group (with challenge and non-supplemented diet) finished at 240 hours post-challenge with a statistically lower survival rate (p<0.05) than the Negative Control group (no challenge and non-supplemented diet), which validates the model used and the bacterial challenge that was carried out. Moreover, although the NF500, AO500 and NF1000 groups showed higher survival rates 240 hours post-challenge than the Positive Control group, this superiority does not reach the level of statistical significance (p>0.05) in any of these cases, as can be seen in the superscript letters. In contrast, the NF500AO500 group combination achieves a much higher survival rate than the Positive Control group and achieves a statistically significant difference (p<0.05), against both the Positive Control group and the NF500 group. In this regard, a synergy against bacterial challenge is shown between the components Nucleoforce^{®} and Aquolive^{®} when combined in a 1:1 ratio (500:500).

## Claims

1. A combined preparation comprising:
a) A first composition comprising a nucleobase and/or a source of nucleobases selected from the group consisting of nucleosides, nucleotides, RNA, DNA, and combinations thereof, and
b) A second composition comprising a mixture of triterpenic acid and polyphenols, wherein the concentration of the first composition relative to the second composition is between 250 and 750 ppm.

2. The combined preparation according to claim 1, wherein the triterpenic acid and polyphenols of the second composition are obtained from a plant extract, preferably, of the Olea genus, more preferably, *Olea europaea.*

3. The combined preparation according to claim 1 or 2, wherein the second composition further comprises fulvic acids.

4. The combined preparation according to any one of claims 1 to 3, wherein the concentration of the first composition relative to the second is between 350 and 650 ppm.

5. The combined preparation according to claim 4, wherein the concentration of the first composition relative to the second is between 450 and 550 ppm.

6. The combined preparation according to claim 5, wherein the concentration of the first composition relative to the second is 500 ppm.

7. A food product comprising a combined preparation according to any one of claims 1 to 6.

8. The food product according to claim 7, wherein the food product is a food, a supplement, an ingredient of a functional food, a medicinal food or a nutraceutical.

9. The food product according to claim 8, wherein the food is a feed, a beverage, a dairy product, a confectionery product, a protein bar, cereals, a cereal bar, a gel or soup.

10. A pharmaceutical composition comprising the combined preparation according to any one of claims 1 to 6.

11. The pharmaceutical composition, according to claim 10, further comprising a pharmaceutically acceptable carrier and/or excipient.

12. The pharmaceutical composition, according to claim 10 or 11, wherein said composition is formulated for oral administration thereof.

13. The combined preparation according to any one of claims 1 to 6, food product according to any one of claims 7 to 9, or pharmaceutical composition according to any one of claims 10 to 12, for use as a medicinal product.

14. The combined preparation according to any one of claims 1 to 6, food product according to any one of claims 7 to 9, or pharmaceutical composition according to any one of claims 10 to 12, for use in improving immunity in a subject.

15. The combined preparation according to any one of claims 1 to 6, food product according to any one of claims 7 to 9, or pharmaceutical composition according to any one of claims 10 to 12, for use in the treatment and/or prevention of a bacterial infection in a subject.

16. The combined preparation, food product or pharmaceutical composition for use according to claim 15, wherein the bacterial infection is caused by *Vibrio parahaemolyticus.*

17. The combined preparation, food product or pharmaceutical composition for use according to any one of claims 14 to 16, wherein the subject is a mammal or an arthropod.

18. The combined preparation, food product or pharmaceutical composition for use according to claim 17, wherein the mammal is a primate, preferably, a human being.

19. The combined preparation, food product or pharmaceutical composition for use according to claim 18, wherein the arthropod is a crustacean, preferably, a caridea, more preferably, a shrimp, a prawn, a baby prawn or a rockpool prawn.
